# EUROPÄISCHE PATENTSCHRIFT

(11) **EP 1 178 032 B1**
(45) Veröffentlichungstag und Bekanntmachung des Hinweises auf die Patenterteilung: **09.04.2003**
(21) Anmeldenummer: 01113460.8
(22) Anmeldetag: 02.06.2001
(51) Int. Cl.: C07C 46/02, C07C 50/20, C07C 49/653, C07C 45/69

(54) **Verfahren zur Herstellung von 2-(4-Methyl-3-pentenyl)-anthrachinon**
Process for the preparation of 2-(4-methyl-3-pentenyl)-anthraquinone
Procédé pour la préparation de 2-(4-méthyl-3-pentényl)-anthraquinone

(30) Priorität: 04.08.2000 DE 10038101
(43) Veröffentlichungstag der Anmeldung: 06.02.2002
(73) Patentinhaber: Degussa AG, 40474 Düsseldorf (DE)
(72) Erfinder: Glenneberg, Jürgen, Dr., 63065 Offenbach (DE); Sauerstein, Holger, 63571 Gelnhausen (DE); Angert, Hubert, Dr., Coqueiral-Aracruz E.S., CEP 29 195-000 (BR)

(56) Entgegenhaltungen:
- EP-A- 0 921 111
- DE-A- 19 816 297
- FR-A- 2 111 190
- US-A- 4 110 353
- PATENT ABSTRACTS OF JAPAN vol. 008, no. 015 (C-206), 21. Januar 1984 (1984-01-21) & JP 58 180452 A (KAWASAKI KASEI KOGYO KK), 21. Oktober 1983 (1983-10-21)

## Beschreibung

Die Erfindung richtet sich auf ein Verfahren zur Gewinnung von 2-(4-Methyl-3-pentenyl)-anthrachinon, nachfolgend als 2-Isohexenylanthrachinon (IHEAQ) bezeichnet durch Oxidation des durch Diels-Alder-Reaktion gebildeten Addukts aus Naphtochinon und Myrcen.

Anthrachinone können gemäß Houben-Weyl, Methoden der organischen Chemie, 4.Auflage, Band VII/3c, Georg Thieme Verlag, Stuttgart 1979, S. 23-31 sowie Band VII/2b, S. 1765 ff hergestellt werden, indem in einer ersten Stufe ein Dien thermisch mit Naphthochinon umgesetzt wird. Diese Diels-Alder-Addition wird üblicherweise in einem Lösungsmittel durchgeführt, kann aber auch direkt durch Erhitzen der Komponenten erfolgen. Sie kann ohne Katalysator aber auch unter Verwendung von Lewis-säuren, wie Bortrifluorid, bewerkstelligt werden.

In einer nachfolgenden Stufe wird das Diels-Alder-Addukt unter Einfluß von Alkalimetallhydroxiden und Luft über die Zwischenstufe der 1,4-Dihydroanthrachinonverbindung zum Anthrachinon umgesetzt. Gemäß den japanischen Offenlegungsschriften JP-A 58-180452 und JP-A 59-51235 lässt sich 2-Isohexenylanthrachinon aus Myrcen und Naphthochinon herstellen, indem das aus diesen Stoffen gebildete Diels-Alder-Addukt in wässrigem Ethanol in Gegenwart eines Alkalihydroxids mit Luft oxidiert wird; das sich bildende gelbe 2-Isohexenylanthrachinon fällt aus dem Reaktionsgemisch aus und lässt sich aus diesem in bekannter Weise isolieren und umkristallisieren.

Das bekannte Verfahren zur Herstellung von 2-Isohexenylanthrachinon (IHEAQ) hat den Nachteil, dass das während der Oxidation sich bildende IHEAQ vor Beendigung der vollständigen Oxidation mit Luft aus der Lösung ausfällt. Dies hat zur Folge, dass sich insbesondere bei der Durchführung des Verfahrens im größeren Maßstab das Reaktionsgemisch nur schwer durchmischen lässt und die Einleitung von Luft oder Sauerstoff behindert und damit die Reaktionszeit bis zum quantitativen Umsatz wesentlich erhöht wird. Zwar lässt sich das Mischproblem beheben, indem eine höhere Lösungsmittelmenge eingesetzt wird, hierdurch wird aber die Raum-Zeit-Ausbeute erniedrigt. Ein weiterer Nachteil des vorbekannten Verfahrens liegt darin, dass ausfallendes IHEAQ nicht umgesetztes Edukt, Zwischenprodukte aber auch Alkalihydroxid einschließt, was zu einem Produkt verminderter Reinheit führt. Die eingeschlossenen Komponenten können bei der Weiterverwendung des IHEAQ, beispielsweise bei der Verwendung desselben als Reaktionsträger im Anthrachinonverfahren zur Herstellung von Wasserstoffperoxid zu Störungen führen; um derartige Störungen zu vermeiden, musste bisher das IHEAQ in aufwendiger Weise umkristallisiert und mit einer Säure gewaschen werden.

Aufgabe der vorliegenden Erfindung ist es demgemäß, ein Verfahren zur Herstellung von 2-Isohexenylanthrachinon aufzuzeigen, das die Probleme des vorgenannten Verfahrens überwindet. Das verbesserte Verfahren sollte einerseits zu einer höheren Raum-Zeit-Ausbeute führen andererseits zu einer höheren Produktreinheit.

Die genannten Aufgaben lassen sich durch das erfindungsgemäße Verfahren lösen. Gefunden wurde demgemäß ein Verfahren zur Herstellung von 2-(4-Methyl-3-pentenyl)-anthrachinon (IHEAQ), umfassend eine Diels-Alder-Addition, wobei Naphtho-1,4-chinon mit Myrcen [7-Methyl-3-methylen-1,6-octadien] umgesetzt wird, und eine Oxidation des gebildeten Diels-Alder-Adduktes [2-(4-Methyl-3-pentenyl)-1,4,11,12-tetrahydroanthrachinon] mit einem Sauerstoff enthaltenden Gas in einem organischen Lösungsmittel in Gegenwart einer Base, das dadurch gekennzeichnet ist, dass man die Oxidation in einem ein polares und ein unpolares organisches Lösungsmittel enthaltenden Lösungsmittelgemisch in Gegenwart einer starken anorganischen und einer organischen Base durchführt. Die Unteransprüche richten sich auf bevorzugte Ausführungsformen des erfindungsgemäßen Verfahrens.

Wie die Beispiele und Vergleichsbeispiele verdeutlichen, lässt sich in der Oxidation der Umsatz des Diels-Alder-Addukts 2-(4-Methyl-3-pentenyl)-1,4,11,12-tetrahydroanthrachinon zur 2'-Isohexenylanthrachinon dadurch steigern, dass eine Kombination aus einer starken anorganischen Base, wie Natriumhydroxid, und einem organischen Amin einsetzt wird. Die durch die Basekombination bewirkte Umsatzsteigerung wird überraschenderweise unabhängig vom Lösungsmittel oder Lösungsmittelgemisch erzielt. Eine besonders hohe Steigerung des Umsatzes lässt sich dadurch bewirken, dass ausser der genannten Basekombination eine Lösungsmittelkombination verwendet wird, welche sowohl ein polares als auch ein unpolares Lösungsmittel enthält. Diese besonders bevorzugte Ausführungsform, also die Oxidation in Gegenwart der genannten Basekombination und Lösungsmittelkombination, führt dazu, dass es während der Oxidation zu keiner Ausfällung kommt, der Umsatz beschleunigt wird und keine unerwünschten Nebenprodukte im 2-Isohexenylanthrachinon eingeschlossen werden.

Die Ursachen der im Stand der Technik vorhandenen Probleme und deren erfindungsgemäße Überwindung lässt sich wie folgt erklären:
Salze auf. Diese sind zwar gut in polaren Lösungsmitteln löslich, aber schlecht in unpolaren. Andererseits sind das Diels-Alder-Addukt und das Endprodukt IHEAQ besser in unpolaren, als in polaren Lösungsmitteln löslich. Starke anorganische Basen sind preiswert, aber durch ihre geringe Löslichkeit in unpolaren Lösungsmittel wenig wirksam. Organische Basen sind teurer, aber sowohl in polaren als auch unpolaren Lösungsmitteln gut löslich. Die Verwendung eines nicht wassermischbaren unpolaren Lösungsmittels erleichtert die nachfolgenden Aufarbeitungsschritte, wie die Abtrennung der Basen durch Auswaschen mit Säure.

Als unpolare Lösungsmittel eignen sich insbesondere aliphatische, cycloaliphatische, aromatische, aromatischaliphatische Kohlenwasserstoffe. Unter den aliphatischen Kohlenwasserstoffen sind verzweigte und unverzweigte Kohlenwasserstoffe mit 6 bis 12 Kohlenstoffatomen, insbesondere 6 bis 10 Kohlenstoffatomen, also Hexane, Heptane, Octane und Decane und Gemische solcher Kohlenwasserstoffe besonders geeignet. Unter den cycloaliphatischen Kohlenwasserstoffen sind Cyclohexan und Terpenkohlenwasserstoffe besonders hervorzuheben. Aus der Reihe der Aromaten und alkylsubstituierten Aromaten sind Benzol und methylierte Benzole, wie Toluol, Xylole, Trimethylbenzole, Tetramethylbenzole und Mischungen solcher methylierter Benzole besonders geeignet; der Benzolring kann anstelle von Methylgruppen oder zusätzlich auch andere niedere Alkylgruppen aufweisen, wie Ethyl, n-Propyl und Isopropyl. Gemische alkylierter, insbesonders methylierter Benzole, sind im Handel erhältlich.

Als polare Lösungsmittel kommen beispielsweise infrage: Alkohole, wie insbesondere Ethanol, n-Propanol, Isopropanol, n-Butanol, Isobutanol, Octanol und Diisobutylcarbinol; Ester, wie insbesondere Essigsäure- und Propionsäureester, beispielsweise Ethylacetat und Methylcyclohexylacetat, Alkylphosphorsäureester, wie Tris-Propionsäureester, beispielsweise Ethylacetat und Methylcyclohexylacetat, Alkylphosphorsäureester, wie Tris-(2-ethylhexyl)-Phosphat; Amide, N-alkylamide, N-alkylpyrrolidone, N,N-Dialkylcarbamate, N-alkylcaprolactame sowie alkylierte Harnstoffe, insbesondere tetraalkylierte Harnstoffe. Bei den zuvor genannten N-alkylierten Verbindungen steht Alkyl vorzugsweise für eine lineare Alkylgruppe mit 1 bis 8 C-Atomen, wobei beispielhaft N-Methylcaprolactam, N-Hexylcaprolactam, N-Octylcaprolactam, N-Methylpyrrolidon, Tetramethyl- und Tetrabutylharnstoff genannt werden.

Das in der bevorzugten Ausführungsform zur verwendende Lösungsmittelgemisch kann ein oder mehrere polare und ein oder mehrere unpolare Lösungsmittel enthalten. Das Mengenverhältnis unpolarer Lösungsmittel zu polaren Lösungsmitteln kann in weiten Grenzen variiert werden; das Gewichtsverhältnis liegt im allgemeinen im Bereich von 5 zu 1 bis 1 zu 5. Vorzugsweise ist der Gewichtsanteil an unpolaren Lösungsmitteln höher als derjenige an polaren Lösungsmitteln; gemäß einer besonders bevorzugten Ausführungsform liegt das Gewichtsverhältnis polarer zu unpolarer Lösungsmittel im Bereich von 1 zu 2 bis 1 zu 4.

Als starke anorganische Basen werden vorzugsweise eine oder mehrere Basen aus der Reihe Lithium-, Natrium- und Kaliumhydroxid oder wässrige Lösungen dieser Hydroxide verwendet.

Bei den zu verwendenden Basen handelt es sich vorzugsweise um stickstoffhaltige Basen. Einsetzbar sind insbesondere primäre, sekundäre und tertiäre aliphatische und cycloaliphatische Amine, wobei es sich hierbei um Mono-, Di- und Triamine handeln kann. Vorzugsweise enthalten die Amine 2 bis 10 Kohlenstoffatome. Beispiele für die genannten Amine sind Ethylamin, Diethylamin, Triethylamin, Mono-, Di- und Tripropyl- oder -butylamin, Ethylendiamin, Diethylentriamin, Morpholin, N-Methylmorpholin, (5.4.0)-undec-7-en). Wirksame organische Basen sind auch Guanidine und Alkylguanidine, wie insbesondere Tetramethylguanidin. Einsetzbar sind auch quaternäre Ammoniumhydroxide und Salze, da letztere in Gegenwart von Alkalihydroxiden in das entsprechende Hydroxid überführt werden; Beispiele sind Tetramethyl und insbesondere Tetrabutylammoniumhydroxid und Salze dieser Basen mit Mineralsäuren.

Pro Mol des Diels-Alder-Addukts werden im allgemeinen 0,01 bis 5 Mol, insbesondere 0, 02 bis 0,5 Mol der Kombination aus organischer und anorganischer Base eingesetzt. Das Mengenverhältnis an anorganischer oder organischer Base ist in weiten Bereichen variabel, üblicherweise wird jedoch die anorganische Base in höherem Anteil eingesetzt. Üblicherweise liegt das Molverhältnis anorganischer Base zu organischer Base im Bereich von 1 zu 4 bis 4 zu 1, insbesondere 1 zu 1 bis 3 zu 1.

Die an sich bekannte Umsetzung Myrcen mit Naphtochinon erfolgt durch Erhitzen der Komponenten in Gegenwart oder Abwesenheit eines Lösungsmittels bei üblicherweise 50 bis 200 °C, vorzugsweise bei 50 bis 120 °C. Bei dieser ersten Stufe können zwar beliebige Lösungsmittel zum Einsatz. kommen, bevorzugt wird jedoch das Lösungsmittelgemisch, das auch in der zweiten Stufe, also der Oxidationsstufe einsetzt wird. Die Produkte Myrcen und 1,4-Naphtochinon werden in stöchiometrischem Verhältnis eingesetzt, jedoch kann Myrcen im Überschuss oder Unterschuss verwendet werden. Die Diels-Alder-Addition wird vorzugsweise in Abwesenheit eines Katalysators durchgeführt, jedoch ist die Mitverwendung eines Katalysators, wie einer Lewis-Säure, beispielsweise Bortrifluorid, möglich.

Das Diels-Alder-Addukt wird, sofern es nicht in Form einer Lösung vorliegt, in einem erfindungsgemäßen Lösungsmittelgemisch enthaltend mindestens ein polares und ein unpolares Lösungsmittel, gelöst. Gemäß einer besonders Lösungsmittelgemisch enthaltend mindestens ein polares und ein unpolares Lösungsmittel, gelöst. Gemäß einer besonders bevorzugten Ausführungsform wird das Lösungsmittelgemisch so zusammengesetzt, dass es ein Azeotrop bildet - hiermit wird die destillative Aufarbeitung wesentlich vereinfacht. Nach Zugabe der erfindungsgemäßen Kombination aus einer anorganischen und einer organischen Base wird die Oxidation des Diels-Alder-Addukts mit einem Sauerstoff enthaltenden Gas, insbesondere Luft oder reinem Sauerstoff, das bei einem Druck von 0,5 bar bis 100 bar (absolut), vorzugsweise im Bereich von 1 bis 10 bar (absolut) in die Lösung eingeleitet wird, durchgeführt. Sauerstoff wird als Oxidationsmittel bevorzugt. Die Oxidation wird bei einer Temperatur im Bereich von 20 bis 200 °C, vorzugsweise 50 bis 150 °C durchgeführt. Die Oxidationsstufe kann in üblichen Reaktionsapparaten, wie beispielsweise einem begasten Rührkessel, einem Schlaufenreaktor oder einer Blasensäule mit und ohne Einbauten durchgeführt werden.

Nach beendeter Umsetzung kann die Base vom Reaktionsgemisch abgetrennt werden, beispielsweise durch Auswaschen mit Wasser und/oder einer Säure oder die Basen werden mittels eines Ionenaustauschers aus dem Reaktionsgemisch abgetrennt. Die weitere Aufarbeitung und Reinigung des 2-Isohexenylanthrachinons kann nach dem Abtrennen des Lösungsmittelgemischs - bevorzugt wird dieses destillativ abgetrennt - durch Umkristallisation und/oder Destillation oder durch Behandlung der Lösung mit einem Adsorptionsmittel, wie Aluminiumoxid oder Aktivkohle, erfolgen.

Das erfindungsgemäße Verfahren zeichnet sich durch eine hohe Raum-Zeit-Ausbeute und hohe Produktreinheit aus. Aufgrund des verbesserten Verfahrens und damit erhaltenen höheren Reinheit des 2-Isohexenylanthrachinons ist es auch möglich, das in dem Lösungsmittelgemisch gelöste IHEAQ direkt einer weiteren Verwendung zuzuführen. Sofern ein üblich ist und IHEAQ ein Reaktionsträger dieses Verfahrens ist, kann das in der erfindungsgemäßen Oxidationsstufe erhaltene Reaktionsgemisch unmittelbar der Arbeitslösung des Anthrachinonprozesses zugeführt werden.

Die nachfolgenden Beispiele und Vergleichsbeispiele verdeutlichen die Durchführung des Verfahrens sowie die dabei erhaltenen Vorteile.

### Beispiele

In einem Doppelmantelgefäß mit Begasungsrührer wurde bei 70 °C eine Menge von 70 mmol des aus 1,4-Naphthochinon und Myrcen gewonnenen Diels-Alder-Addukts 2-(4-Methyl-3-pentenyl)-1,4,11,12-tetrahydroanthrachinon in 80 ml Lösungsmittel beziehungsweise Lösungsmittelgemisch gelöst; die angegebene Base beziehungsweise Basekombination wurde zugegeben und die Lösung mit Sauerstoff 1 h oxidiert. Die Reaktionsmischung wurde mit HPLC analysiert. Die Einsatzmengen und Raum-Zeit-Ausbeute folgen aus der Tabelle. Die Zahl hinter dem Lösungsmittel gibt den Volumenanteil in der Lösungsmittelmischung an. Die Zahl hinter der Base gibt die eingesetzte Menge in mmol an.

Bei allen Versuchen traten während der Umsetzung mit Base keine Ausfällungen auf und das Reaktionsgemisch blieb auch nach Abkühlung auf Raumtemperatur flüssig.

**Tabelle**

| Beispiel Nr. | Unpolares Lösungsmittel | Polares Lösungsmittel | Anorganische Base | Organische Base | Umsatz an Diels-Alder-Addukt zu IHEAQ nach 1 h Reaktionszeit (Mol-%) |
|---|---|---|---|---|---|
| 1 *) | Toluol 100 | - | - | Di-Etamin 7 | 36 |
| 2 *) | Toluol 100 | - | NaOH 14 | - | 2 |
| 3 *) | Toluol 100 | - | NaOH 14 | Di-Etamin 7 | 57 |
| 4 *) | Toluol 75 | n-Butanol 25 | NaOH 14 | - | 35 |
| 5 *) | Toluol 75 | n-Butanol 25 | - | Di-Etamin 7 | 58 |
| 6 **) | Toluol 75 | n-Butanol 25 | NaOH 14 | Di-Etamin 7 | 100 |

| | | | | | |
|---|---|---|---|---|---|
| *) nicht erfindungsgemäßes Beispiel | | | | | |
| **) erfindungsgemäßes Beispiel | | | | | |

### Beispiel 7 (nicht erfindungsgemäß)

In reinem n-Butanol als Lösungsmittel wurden 70 mmol Diels-Alder-Addukt und 14 mmol NaOH (als 3,5 mmol wässrige Lösung) wie oben beschrieben mit Sauerstoff umgesetzt. Das Diels-Alder-Addukt war zwar schon nach kurzer Zeit zum IHEAQ umgesetzt, es fiel aber ein flockiger Niederschlag aus. Das Reaktionsgemisch wurde beim Abkühlen auf unter 60 °C fest und ließ sich nur schwer aufarbeiten.

### Beispiel 8

Nachfolgend die derzeit beste Ausführungsform des Gesamtverfahrens

In einer Mischung aus 4000 ml Toluol und 1300 ml n-Butanol werden 850 g Naphtochinon (95 %ig) vorgelegt und unter Erhitzen auf 90 °C insgesamt 990 g Myrcen (79 %ig) zugegeben. Nach 5 h ist die Reaktion zum Diels-Alder-Addukt abgeschlossen. Durch HPLC wird eine Ausbeute an Diels-Alder-Addukt (bezogen auf eingesetztes Naphtochinon) von 92 % bestimmt.

Die Lösung des Diels-Alder-Addukts wird auf 70 °C abgekühlt; dann werden 250 ml Wasser, 52 ml 50 %ige Natronlauge und 50 ml Diethylamin zugegeben. Die Lösung wird anschließend mit Sauerstoff bei 70 °C begast. Nach 5 h ist die Sauerstoffaufnahme beendet. Eine HPLC-Messung zeigt vollständigen Umsatz zum IHEAQ [Die wässrige Phase wird abgetrennt, die organische Phase mit Wasser und verdünnter Phosphorsäure ausgewaschen]. Die Lösungsmittel werden abdestilliert, der Rückstand an IHEAQ durch Destillation gereinigt. Erhalten wird reines IHEAQ in 87 %iger Ausbeute und über 96 %iger Reinheit.

## Patentansprüche

1. Verfahren zur Herstellung von 2-(4-Methyl-3-pentenyl)-anthrachinon (IHEAQ), umfassend eine Diels-Alder-Addition, wobei Naphtho-1,4-chinon mit Myrcen [7-Methyl-3-methylen-1,6-octadien] umgesetzt wird, und eine Oxidation des gebildeten Diels-Alder-Adduktes [2-(4-Methyl-3-pentenyl)-1,4,11,12-tetrahydroanthrachinon] mit einem Sauerstoff enthaltenden Gas in einem organischen Lösungsmittel in Gegenwart einer Base,
**dadurch gekennzeichnet,**
**dass** man die Oxidation in einem ein polares und ein unpolares organisches Lösungsmittel enthaltenden Lösungsmittelgemisch in Gegenwart einer starken anorganischen und einer organischen Base durchführt.

2. Verfahren nach Anspruch 1,
**dadurch gekennzeichnet,**
**dass** man als unpolares Lösungsmittel einen aliphatischen, cycloaliphatischen, aromatischen und aromatisch-aliphatischen Kohlenwasserstoff verwendet.

3. Verfahren nach Anspruch 2,
**dadurch gekennzeichnet,**
**dass** man als unpolares Lösungsmittel ein alkyliertes Benzol aus der Reihe Toluole, Xylole, Trimethylbenzole, Tetramethylbenzole und Mischungen von Alkylbenzolen verwendet.

4. Verfahren nach einem der Ansprüche 1 bis 3,
**dadurch gekennzeichnet,**
**dass** man als polares Lösungsmittel ein solches aus der Reihe der Alkohole, Ester, Phosphorsäureester, Amide, N-Alkylamide, N-Alkylpyrrolidone, N,N-Dialkylcarbamate, alkylierten Harnstoffen und N-Alkylcaprolactamen verwendet.

5. Verfahren nach Anspruch 4,
**dadurch gekennzeichnet,**
**dass** man als polares Lösungsmittel ein solches aus der Reihe, Ethanol, n-Propanol, n-Butanol, Octanol, Diisobutylcarbinol, Methylcyclohexylacetat, Tris-(2-ethylhexyl)-phosphat, Tetramethyl- und Tetrabutylharnstoff, N-Methylpyrrolidon und N-Methyl-, Hexyl- und Octylcaprolactam verwendet.

6. Verfahren nach einem der Ansprüche 1 bis 5,
**dadurch gekennzeichnet,**
**dass** man als anorganische Base Lithium-, Natrium- oder Kaliumhydroxid oder ein Gemisch hiervon oder eine wässrige Lösung der Alkalihydroxide verwendet.

7. Verfahren nach einem der Ansprüche 1 bis 6,
**dadurch gekennzeichnet,**
**dass** man als organische Base eine solche aus der Reihe primärer, sekundärer und tertiärer aliphatischer und cycloaliphatischer Mono-, Di- und Triamine, Tetraalkylammoniumhydroxiden und -salzen, Tetramethylguanidin, 1,8-Diazabicyclo-(5.4.0)-undec-7-en und 1,4-Diaza-bicyclo-2,2,2-octan verwendet.

8. Verfahren nach einem der Ansprüche 1 bis 7,
**dadurch gekennzeichnet,**
**dass** man ein oder mehrere polare und ein oder mehrere unpolare Lösungsmittel im Gewichtsverhältnis im Bereich von 5 zu 1 bis 1 zu 5, insbesondere 1 zu 2 bis 1 zu 4, einsetzt.

9. Verfahren nach einem der Ansprüche 1 bis 8,
**dadurch gekennzeichnet,**
**dass** man pro Mol des Diels-Alder-Addukts 0,01 bis 5 Mol, insbesondere 0,02 bis 0,5 Mol, der Kombination aus organischer und anorganischer Base einsetzt.

10. Verfahren nach einem der Ansprüche 1 bis 9,
**dadurch gekennzeichnet,**
**dass** man anorganische Base und organische Base im Molverhältnis im Bereich von 1 zu 4 bis 4 zu 1, insbesondere 1 zu 1 bis 3 zu 1, einsetzt.

11. Verfahren nach einem der Ansprüche 1 bis 10,
**dadurch gekennzeichnet,**
**dass** man die Oxidation bei 20 bis 200 °C, insbesondere 50 bis 150 °C bei einem Druck des eingeleiteten Gases im Bereich von 1 bis 10 bar (absolut) unter Einsatz von Sauerstoff durchführt.

## Claims

1. Process for the preparation of 2-(4-methyl-3-pentenyl)anthraquinone (IHEAQ), comprising a Diels-Alder addition, wherein naphtho-1,4-quinone is reacted with myrcene [7-methyl-3-methylene-1,6-octadiene], and an oxidation of the resulting Diels-Alder adduct [2-(4-methyl-3-pentenyl)-1,4,11,12-tetrahydroanthraquinone] using an oxygen-containing gas in an organic solvent in the presence of a base,
**characterised in that**
the oxidation is carried out in a solvent mixture containing a polar and a non-polar organic solvent, in the presence of a strong inorganic base and of an organic base.

2. Process according to claim 1,
**characterised in that**
the non-polar solvent used is an aliphatic, cycloaliphatic, aromatic or aromatic-aliphatic hydrocarbon.

3. Process according to claim 2,
**characterised in that**
the non-polar solvent used is an alkylated benzene from among the toluenes, xylenes, trimethylbenzenes, tetramethylbenzenes and mixtures of alkylbenzenes.

4. Process according to one of claims 1 to 3,
**characterised in that**
the polar solvent used is such a solvent from among the alcohols, esters, phosphoric esters, amides, N-alkylamides, N-alkylpyrrolidones, N,N-dialkyl carbamates, alkylated ureas and N-alkylcaprolactams.

5. Process according to claim 4,
**characterised in that**
the polar solvent used is such a solvent from among ethanol, n-propanol, n-butanol, octanol, diisobutylcarbinol, methyl cyclohexylacetate, tris(2-ethylhexyl) phosphate, tetramethyl- and tetrabutylurea, N-methylpyrrolidone and N-methyl, hexyl- and octylcaprolactam.

6. Process according to one of claims 1 to 5,
**characterised in that**
the inorganic base used is lithium hydroxide, sodium hydroxide or potassium hydroxide or a mixture of these or an aqueous solution of the alkali hydroxides.

7. Process according to one of claims 1 to 6,
**characterised in that**
the organic base used is such a base from among the primary, secondary and tertiary aliphatic and cycloaliphatic mono, di- and triamines, tetraalkylammonium hydroxides and tetraalkylammonium salts, tetramethylguanidine,
1,8-diazabicyclo[5.4.0]undec-7-ene and
1,4-diazabicyclo[2.2.2]octane.

8. Process according to one of claims 1 to 7,
**characterised in that**
one or more polar and one or more non-polar solvents are used in the weight ratio in the range of 5 to 1 up to 1 to 5, in particular 1 to 2 up to 1 to 4.

9. Process according to one of claims 1 to 8,
**characterised in that**
0.01 to 5 mol, in particular 0.02 to 0.5 mol, of the combination of organic and inorganic base is used per mol of the Diels-Alder adduct.

10. Process according to one of claims 1 to 9,
**characterised in that**
inorganic base and organic base are used in the molar ratio in the range of 1 to 4 up to 4 to 1, in particular 1 to 1 up to 3 to 1.

11. Process according to one of claims 1 to 10,
**characterised in that**
the oxidation is carried out using oxygen at 20 to 200°C, in particular 50 to 150°C, the introduced gas being at a pressure in the range of 1 to 10 bar (absolute).

## Revendications

1. Procédé pour la préparation de la 2-(4-méthyl-3-pentényl)-anthraquinone (IHEAQ), comprenant une addition de Diels-Alder dans laquelle on fait réagir de la naphto-1,4-quinone avec du myrcène (7-méthyl-3-méthylène-1,6-octadiène), et une oxydation de l'adduit de Diels-Alder formé [2-(4-méthyl-3-pentényl)-1,4,11,12-tétrahydroanthraquinone] avec un gaz contenant de l'oxygène, dans un solvant organique, en présence d'une base,
**caractérisé en ce qu'**
on effectue l'oxydation dans un mélange de solvants contenant un solvant organique polaire et un solvant organique non polaire, en présence d'une base organique et d'une base non organique forte.

2. Procédé selon la revendication 1,
**caractérisé en ce qu'**
on utilise en tant que solvant non polaire un hydrocarbure aliphatique, cycloaliphatique, aromatique ou aromatique-aliphatique.

3. Procédé selon la revendication 2,
**caractérisé en ce qu'**
on utilise comme solvant non polaire un benzène alkylé choisi parmi des toluènes, xylènes, triméthylbenzènes, tétraméthylbenzènes et des mélanges d'alkylbenzènes.

4. Procédé selon l'une quelconque des revendications 1 à 3,
**caractérisé en ce qu'**
on utilise comme solvant polaire un solvant polaire choisi dans l'ensemble contenant des alcools, des esters, des esters d'acide phosphorique, des amides, des N-alkylamides, des N-alkylpyrrolidones, des N,N-dialkylcarbamates, des urées alkylées et des N-alkylcaprolactames.

5. Procédé selon la revendication 4,
**caractérisé en ce qu'**
on utilise comme solvant polaire un solvant polaire choisi dans le groupe constitué par l'éthanol, le n-propanol, le n-butanol, l'octanol, le diisobutylcarbinol, le cyclohexylacétate de méthyle, le phosphate de tris-(2-éthylhexyle), la tétraméthylurée et la tétrabutylurée, la N-méthylpyrrolidone et le N-méthyl-, hexyl- et octylcaprolactame.

6. Procédé l'une quelconque des revendications 1 à 5,
**caractérisé en ce qu'**
on utilise comme base non organique l'hydroxyde de lithium, sodium, ou potassium ou un mélange de ceux-ci, ou une solution aqueuse des hydroxydes de métaux alcalins.

7. Procédé selon l'une quelconque des revendications 1 à 6,
**caractérisé en ce qu'**
on utilise comme base organique une base organique choisie dans le groupe constitué par les mono- di- et triamines aliphatiques et cycloaliphatiques primaires, secondaires et tertiaires, les hydroxydes de tétraalkylammonium et leurs sels, la tétraméthylguanidine, le 1,8-diazabicyclo(5.4.0)undéc-7-ène et le 1,4-diazabicyclo-2,2,2-octane.

8. Procédé selon l'une quelconque des revendications 1 à 7,
**caractérisé en ce qu'**
on utilise un ou plusieurs solvants polaires et un ou plusieurs solvants non polaires en proportion pondérale dans la plage allant de 5:1 à 1:5, en particulier de 1:2 à 1:4.

9. Procédé selon l'une quelconque des revendications 1 à 8,
**caractérisé en ce qu'**
on utilise, par mole de l'adduit de Diels-Alder, de 0,01 à 5 moles, en particulier de 0,02 à 0,5 mole, de l'association de base organique et de base non organique.

10. Procédé selon l'une quelconque des revendications 1 à 9,
**caractérisé en ce qu'**
on utilise la base non organique et la base organique en un rapport molaire dans la plage allant de 1:4 à 4:1, en particulier de 1:1 à 3:1.

11. Procédé selon l'une quelconque des revendications 1 à 10,
**caractérisé en ce qu'**
on effectue l'oxydation à une température de 20 à 200°C, en particulier de 50 à 150°C, sous une pression du gaz introduit dans la plage de 1 à 10 bars (pression absolue), en utilisant de l'oxygène.
